(19) Europäisches Patentamt
European Patent Office
Office européen des brevets

(11) Veröffentlichungsnummer: **0 282 792**
**B1**

(12) # EUROPÄISCHE PATENTSCHRIFT

(45) Veröffentlichungstag der Patentschrift: **30.05.90**

(51) Int. Cl.⁵: **C07D 303/32,** A01N 43/20

(21) Anmeldenummer: **88102906.0**

(22) Anmeldetag: **26.02.88**

(54) **Substituierte 2,3-Epoxy-5-cyclohexenonderivate und diese enthaltende Fungizide und Herbizide.**

(30) Priorität: **07.03.87 DE 3707358**

(43) Veröffentlichungstag der Anmeldung:
**21.09.88 Patentblatt 88/38**

(45) Bekanntmachung des Hinweises auf die Patenterteilung:
**30.05.90 Patentblatt 90/22**

(84) Benannte Vertragsstaaten:
**DE FR GB IT**

(56) Entgegenhaltungen:
**CHEMISCHE BERICHTE, Band 93, Nr. 8, 8 August 1960, Seiten 1896-1899, Weinheim, DE; K. ALDER et al.: "Darstellung von p-Chinon-epoxyden"**
**AGRICULTURAL AND BIOLOGICAL CHEMISTRY, Band 39, Nr. 2, 1975, Seiten 555-556, Tokyo, JP; A. ICHIHARA et al.: "Synthesis of 7-Desoxypanepoxydol"**
**JOURNAL OF THE CHEMICAL SOCIETY, PERKIN TRANSACTIONS I, Nr. 8, August 1987, Seiten 1861-1864, London, GB; J.C. BLAZEJEWSKI et al.: "Chlorous acid oxidation of trifluoromethylphenols: Cyclopentenolones by benzilic acid ring contraction"**
**AGRICULTURAL AND BIOLOGICAL CHEMISTRY, Band 43, Nr. 1, 1979, Seiten 113-116, Tokyo, JP; H. HAYASHI et al.: "Methoxy-1, 4-benzoquinone and methyl gallate, inhibitory factors of betacyanin synthesis in Amaranthus, from Persimmon fruits"**

(73) Patentinhaber: **BASF Aktiengesellschaft, Carl-Bosch-Strasse 38, D-6700 Ludwigshafen(DE)**

(72) Erfinder: **Wriede, Ulrich, Dr., Albert-Einstein-Allee 10, D-6703 Limburgerhof(DE)**
Erfinder: **Speakman, John-Bryan, Dr., U 4,2, D-6800 Mannheim 1(DE)**
Erfinder: **Karl, Rudolf, Dr., Muehlweg 22, D-6703 Limburgerhof(DE)**
Erfinder: **Pommer, Ernst-Heinrich, Dr., Berliner Platz 7, D-6703 Limburgerhof(DE)**
Erfinder: **Ammermann, Eberhard, Dr., Sachsenstrasse 3, D-6700 Ludwigshafen(DE)**
Erfinder: **Wuerzer, Bruno, Dr., Ruedigerstrasse 13, D-6701 Otterstadt(DE)**

(56) Entgegenhaltungen: (Fortsetzung)
**CHEMICAL ABSTRACTS, Band 100, Nr. 9, 27 Februar 1984, Seite 206, Zusammenfassung Nr. 63508a, Columbus, Ohio, US; & JP-A-58 174 304 (TEIJIN LTD) 13-10-1983**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-substituierte 2,3-Epoxy-5-cyclohexenonderivate, Verfahren zu ihrer Herstellung sowie ihre Verwendung als Fungizide und Herbizide.

Es ist bekannt, N-Trichlormethylthio-tetrahydrophthalimid als Fungizid zu verwenden (Wegler, Chemie der Pflanzenschutz- und Schädlingsbekämpfungsmittel, Band 2, Seite 109, (1970).

Es ist ferner die Verbindung 2-Hydroxymethyl-2,3-epoxy-5-cyclohexen-1,4-dion bekannt (JP-OS 83/174 304).

Es wurde nun gefunden, daß 2-substituierte 2,3-Epoxy-5-cyclohexenonderivate der Formel I,

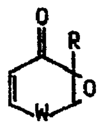

(I)

in welcher die Substituenten folgende Bedeutung haben
W C=O oder $CHOR^1$,
$R^1$ Wasserstoff, $C_1$-$C_4$-Alkyl, $C_2$-$C_4$-Alkenyl, $C_2$-$C_4$-Alkinyl oder $C_2$-$C_4$-Alkylcarbonyl
R eine geradkettige oder verzweigte, gesättigte oder ungesättigte Kohlenwasserstoffkette mit 1 bis 10 C-Atomen, die gegebenenfalls durch Halogen substituiert ist oder den Rest

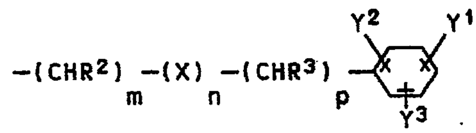

.

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl, $C_1$-$C_4$-Alkylthio, $C_1$-$C_4$-Halogenalkoxy, $C_1$-$C_4$-Halogenalkylthio, $C_1$-$C_4$-Alkylsulfonyl, Halogen, Cyano, Nitro, gegebenenfalls substituiertes Phenyl oder gegebenenfalls substituiertes Phenoxy
m, p unabhängig voneinander 0, 1, 2 oder 3
n 0 oder 1
eine gute fungizide und herbizide Wirksamkeit und Pflanzenverträglichkeit besitzen.

Unter den Verbindungen I sind diejenigen bevorzugt, in denen die Substituenten beispielsweise folgende Bedeutung haben:
$R^1$ Wasserstoff
$C_1$-$C_4$-Alkyl, besonders Methyl, Ethyl, iso-Propyl, $C_2$-$C_4$-Alkenyl, besonders Allyl, $C_2$-$C_4$-Alkylcarbonyl, besonders Acetyl
R eine $C_1$-$C_{10}$-geradkettige oder verzweigte, gesättigte oder ungesättigte, gegebenenfalls durch Halogen (Fluor, Chlor, Brom) substituierte Kohlenwasserstoffkette, insbesondere $C_1$-$C_6$-Alkyl, besonders Methyl, Ethyl, n-Propyl, iso-Propyl, n-Butyl, 1-Methylpropyl, tert.-Butyl, n-Pentyl, 3-Methylbutyl, Neopentyl, n-Hexyl, insbesondere $C_3$-$C_5$-Alkenyl, Allyl, 3-Methylallyl, 2-Methylallyl, 3-Butenyl, 3-Methyl-2-butenyl, insbesondere $C_3$-$C_5$-Alkinyl, Propargyl, 2-Butinyl, 3-Methyl-3-buten-1-inyl, 3-Methyl-3-penten-1-inyl, 2,3-Dibrompropyl, 3-Chlor-3-methyl-butyl, 3-Brom-3-methylbutyl, 2,3-Dibrom-3-methylbutyl, 3-Trifluormethyl-3-buten-1-inyl, oder den Rest

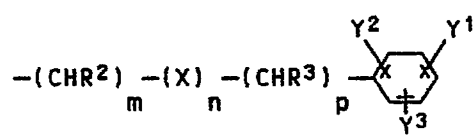

$Y^1$, $Y^2$ und $Y^3$ Wasserstoff,
$C_1$-$C_4$-Alkyl, besonders 2-, 3- oder 4-Methyl, 4-tert. Butyl,
$C_1$-$C_4$-Alkoxy, besonders 2-, 3- oder 4-Methoxy,
$C_1$-$C_4$-Halogenalkyl, besonders 3-oder 4-Trifluormethyl,
$C_1$-$C_4$-Alkylthio, besonders 4-Methylthio,
$C_1$-$C_4$-Alkylsulfonyl, besonders 4-Methylsulfonyl,
Halogen, besonders 3- oder 4-Fluor, 2-, 3- oder 4-Chlor, 3-Brom, 2,4- oder 3,4-Dichlor, 2,4,5-Trichlor, 2,4-Dibrom
Cyano, besonders 3- oder 4-Cyano
Nitro, besonders 2-, 3- oder 4-Nitro
Phenyl, $C_1$-$C_4$-Alkylphenyl, Halogenphenyl, Phenoxy, $C_1$-$C_4$-Alkylphenoxy oder Halogenphenoxy,

und R insbesondere die Reste: Phenyl, Benzyl, 1-Phenylethyl, Phenoxymethyl, 1-Phenoxyethyl, Phenylthiomethyl, Benzyloxy, 1-Phenylethoxy, 3-(Benzyloxy)propyl, 3-(1-Phenylethoxy)propyl, 3-Phenoxypropyl, 3-Phenylthiopropyl bedeutet.

Weiterhin wurde gefunden, daß man die neuen, 2-substituierten 2,3-Epoxy-5-cyclohexenonderivate der Formel I dadurch erhält, daß man 2-substituierte Benzochinone (Ann. Chem. 763, 135 (1972), Chem. Ber. 97, 1926 (1964), Agr. Biol. Chem. 39, 555 (1975)) der Formel II in Gegenwart oder Abwesenheit eines Lösungsmittels an Cyclopentadien in etwa stöchiometrischen Mengen zu den Verbindungen III cycloaddiert (Chem. Ber. 90, 1896 (1960)).

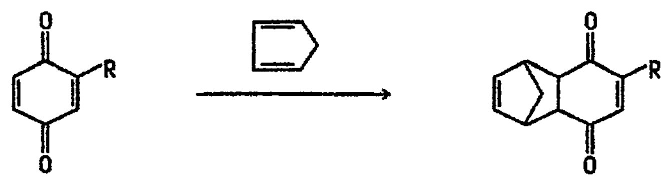

II III

Als Lösungsmittel sind aromatische Kohlenwasserstoffe, beispielsweise Benzol, Toluol, Xylol, chlorierte Kohlenwasserstoffe, z.B. Methylenchlorid, Chloroform, Tetrachlorkohlenstoff, 1,2-Dichlorethan, 1,1,1-Trichlorethan, Ether, z.B. Diethylether, Diisopropylether, Methyl-tert.-butylether, Tetrahydrofuran, Dioxan, Ethylenglycolether, Carbonsäureester, z.B. Ameisensäureethylester, Essigsäureethylester, Propionsäureethylester, Ketone, z.B. Aceton, Methylethylketon, Amide, z.B. Formamid, Dimethylformamid, Alkohole, z.B. Methanol, Ethanol, n-, iso-Propanol, n-, iso- oder tert.-Butanol geeignet; bevorzugt werden Methanol und Ethanol. Die Reaktion kann im Temperaturbereich von -50 bis 40°C durchgeführt werden, vorzugsweise bei 0 bis 20°C.

Die Verbindung III wird durch Epoxidierung in die Verbindung IV überführt (Houben-Weyl VI/3 S. 397 ff.).

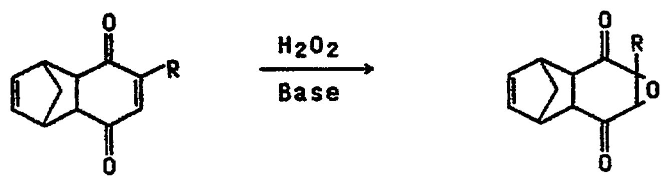


III IV

Als Peroxoverbindungen, die z.B. in stöchiometrischen Mengen oder bis zum 20fachen Überschuß eingesetzt werden können, eignen sich beispielsweise Wasserstoffperoxid, Natriumperborat, Natriumhypochlorit (NaOCl) oder t-Butylhydroperoxid. Als Basen kommen in Betracht Alkalihydroxide, z.B. Lithiumhydroxid, Natriumhydroxid oder Kaliumhydroxid, Alkalicarbonate, z.B. Natriumcarbonat oder Kaliumcarbonat, organische Ammoniumhydroxide, z.B. Benzyltrimethylammoniumhydroxid, Tetraethylammoniumhydroxid, Tetramethylammoniumhydroxid oder ein basischer Ionenaustauscher. Als Lösungsmittel können neben Wasser bevorzugt Alkohole, wie Methanol, Ethanol, n-, iso-Propanol, n-, iso-oder tert.-Butanol, Ether, wie Tetrahydrofuran, Dioxan oder Ethylenglycolether oder Amide wie Formamid, Dimethylformamid Verwendung finden. Bevorzugt wird Tetrahydrofuran. Die Reaktion wird z.B. im Temperaturintervall von -30 bis -50°C durchgeführt, vorzugsweise bei -10 bis +20°C.

Die Verbindung IV wird (Chem. Ber. 93, 1896 (1963)) den Bedingungen der Thermolyse unterworfen, wobei Ia gebildet wird.

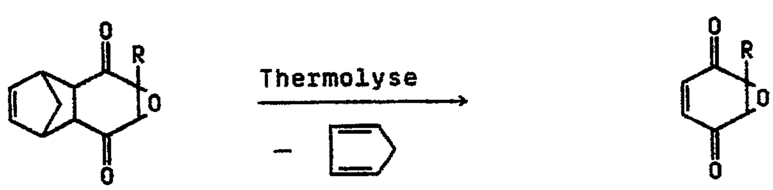


IV Ia

Die Verbindung IV wird z.B. bei 150 bis 350°C und Drucken von 0,001 bis 0,1 Torr durch einen auf 300 bis 500°C, bevorzugt bei 400 bis 450°C vorgeheizten Ofen in kurzer Zeit durchgeleitet und pyrolysiert.

Die Verbindung Ia wird gegebenenfalls zur Verbindung Ib ($R^1$ = H) reduziert.

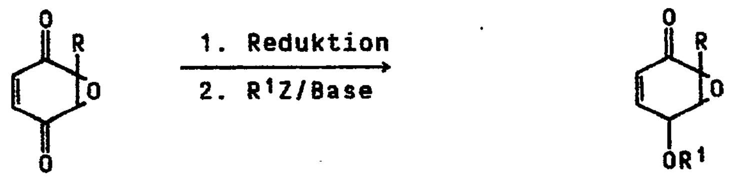


Für die Reduktion sind Hydridverbindungen beispielsweise Lithiumborhydrid, Lithiumcyanoborhydrid, Lithiumtrialkylborhydride, Natriumborhydid, Natriumcyanoborhydrid, Natriumtrialkylborhydride, Natriumtrimethoxyborhydrid, Natriumtriacetoxyborhydrid, Kaliumborhydrid, Natriumtrithiodihydridoboranat; Tetraalkylammoniumborhydride, Tetraalkylammoniumcyanoborhydride, Magnesiumborhydrid, Calciumborhydrid, Zinkborhydrid, Diboran, Bis(trifluoracetoxy)boran, Bis(triphenylphosphan)-kupfer(I)borhydrid, Tetracis(triphenylphosphan)-dikupfer(I)dicyano-hexahydridoboranat, Trialkylzinnhydride, Natriumhydrid oder Alkylsiliciumhydride geeignet. Auch Metalle wie Zink oder Eisen in Gegenwart von Säuren können verwendet werden. Bevorzugt als Reduktionsmittel wird Natriumcyanoborhydrid.

Als Lösungsmittel kommen in Frage: Kohlenwasserstoffe, z.B. Petrolether, Cyclohexan, Ligroin, Decalin, Benzol, Toluol, Xylol, chlorierte Kohlenwasserstoffe, z.B. Methylenchlorid, Dichlorbutan, Chloroform, Tetrachlorkohlenstoff, 1,1,1-Trichlorethan, Ether, z.B. Diethylether, Methyl-tert.-Butylether, Diisopropylether, Ethylenglycoldimethylether, Tetrahydrofuran, Dioxan, Amide wie Formamid, Methylformamid, Dimethylformamid, Alkohole wie Methanol, Ethanol, n-, iso-Propanol, n-, tert.-Butanol und entsprechende Gemische. Bevorzugt werden Tetrahydrofuran oder Methanol. Die Reaktion wird wegen der Basenlabilität der Verbindung Ia in neutralem bis schwach saurem Milieu (pH 3 -7) in einem Temperaturbereich von -30 bis +40°C, vorzugsweise bei -10 bis +20°C durchgeführt und chromatographisch kontrolliert, bis kein Ausgangsprodukt mehr vorhanden ist. Als Säurezusatz der zumeist alkalisch reagierenden Reduktionsmitttel kommen neben verd. Mineralsäure wie Salzsäure, Schwefelsäure, Phosphorsäure, Carbonsäuren wie Ameisensäure, Essigsäure, Propionsäure , Trifluoressigsäure, Chloressigsäure, Oxalsäure sowie sauer eingestellte Puffersysteme in Frage. Bevorzugt wird Essigsäure. Für Reaktionen in aprotischem Medium können Lewis-Säuren wie Aluminiumchlorid, Eisen-III-chlorid, Zinkchlorid oder Bortrifluoridetherat verwendet werden.

Gegebenenfalls wird die Verbindung Ib ($R^1$ = H) zur Verbindung Ib ($R^1$ verschieden von H) umgesetzt.

Die Alkylierung oder Acylierung der Verbindung Ib kann in Gegenwart eines Säureaceptors mit einem Alkylierungs- oder Acylierungsmittel der Formel $R^1Z$ erfolgen, wobei Z eine leicht abspaltbare Gruppierung z. B. Chlorid, Bromid, Iodid, Tosylat, Mesylat, Triflat oder Acetat bedeutet. Als Säureakzeptoren dienen vorzugsweise tertiäre Amine, Erdalkaliverbindungen, Ammoniumverbindungen und Alkalimetallverbindungen sowie entsprechende Gemische. Es kommen z. B. folgende basischen Verbindungen in Betracht: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkhydrogencarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Pyridin, α,β,γ-Picolin, 2,6-Lutidin, Pyrimidin, N,N-Dimethylanilin, N,N-Diethylanilin, N-Methylpyrrolidon oder N-Ethylpyrrolidon.

Die Reaktion kann in Gegenwart oder Abwesenheit eines Lösungsmittels im Temperaturbereich zwischen 20 und 150°C durchgeführt werden. Als Lösungsmittel sind geeignet: arom. Kohlenwasserstoffe, wie Benzol, Toluol, Xylole, chlorierte Kohlenwasserstoffe, wie Methylenchlorid, Chloroform, Tetrachlorkohlenstoff, 1,2-Dichlorethan, 1,1,1-Trichlorethan, Chlorbenzol, Ether, wie Diethylether, Diisopropylether, Methyl-tert.-Butylether, Tetrahydrofuran, Dioxan, Ethylenglycolether, Ketone, wie Aceton, Methylethylketon, Cyclohexanon, Amide wie Formamid, Methylformamid, Dimethylformamid, N-Methyl-pyrolidon, N-Ethylpyrolidon oder Nitrile, wie Acetonitril.

Die Verbindung Nr. 169 (2,3-Epoxy-2-(3-methyl-3-buten-1-inyl)-5-cyclohexen-4-ol-1-on = 1-(3-Methyl-3-buten-1-inyl)-5-hydroxy-7-oxabicyclo[4.1.0]hept-3-en-2-on) kann wie oben beschrieben hergestellt werden. Sie kann aber auch durch Kultivierung des Pilzes DSM 3650 und Abtrennung aus dem Kulturfiltrat hergestellt werden.

Der Pilz DSM 3650 wurde von befallenen Maiswurzeln isoliert, die in der Bundesrepublik Deutschland gefunden worden sind (Krüger, W. und Speakman, J.B.; Zeitschrift für Pflanzenkrankheiten und Pflanzenschutz, 1984, 91 (1), 1-11).

Eine lebensfähige Kultur dieses Mikroorganismus wurde bei der Deutschen Sammlung für Mikroorganismen, Grisebachstraße 8, D-3400 Göttingen, hinterlegt und deren permanenter Sammlung einverleibt. Der Mikroorganismus ist der Öffentlichkeit bei dieser Hinterlegungsstelle unter seiner Hinterlegungsnummer DSM 3650 frei zugänglich.

Die Verbindung Nr. 169 wird, allgemein gesprochen, dadurch hergestellt, daß man den Pilz DSM 3650 unter aeroben Bedingungen in einem flüssigen Medium, enthaltend assimilierbare Quellen an Kohlenstoff, Stickstoff und anorganischen Anionen und Kationen, kultiviert, bis sich eine wesentliche Menge der Verbindung Nr. 169 in dem Medium gebildet hat und nachfolgend diese Verbindung daraus gewinnt.

Die Verbindung Nr. 169 wird beispielsweise hergestellt, indem man unter aeroben Bedingungen ein flüssiges Medium fermentiert, das assimilierbare Quellen an Kohlenstoff, Stickstoff und anorganischen Anionen und Kationen enthält, wobei das Medium mit einer lebenfähigen Kultur des Pilzes DSM 3650 oder einer die Verbindung Nr. 169 produzierenden Mutante desselben beimpft wurde, den Organismus bei steriler Belüftung und unter Rühren während eines Zeitraumes von 50 bis 150 Stunden bei einer Temperatur von 20 bis 30°C und pH 5,5 bis 8,0 kultiviert, das Kulturfiltrat abtrennt und die Verbindung daraus extrahiert.

Die Extraktion der Verbindung Nr. 169 kann beispielsweise in der Weise erfolgen, daß man sie aus dem Kulturfiltrat mit einem mit Wasser nicht mischbaren Lösemittel oder nach Gefriertrocknung des Kulturfiltrates mit einem Lösemittel aus diesem extrahiert.

Die Reinigung des Extraktes kann beispielsweise durch chromatographische Verfahren erfolgen.

Für die Anwendung als Fungizid oder Herbizid ist nicht nur die Verbindung Nr. 169 als solche, sondern auch die Fermentationsbrühe oder Gesamtmaische des Mikroorganismus DSM 3650 geeignet.

Man kann auch den aus der Fermentationsbrühe oder Gesamtmaische des Mikroorganismus DSM 3650 gewonnenen Extrakt verwenden.

Die Verbindung Nr. 169 entsteht im Verlauf der Kultivierung des Pilzes DSM 3650 unter kontrollierten Bedingungen.

Allgemeine Fermentationsbedingungen

Die Kultivierung des Pilzes DSM 3650 kann in einer breiten Vielfalt flüssiger Kulturmedien durchgeführt werden. Geeignete Nährmedien umfassen eine assimilierbare Quelle von Kohlenstoff, wie Dextrin, Saccharose, Melasse, Glycerin, etc.; eine assimilierbare Quelle an Stickstoff, wie Protein, Proteinhydrolysat, Polypeptide, Aminosäure, Maisquellwasser, etc.; sowie anorganische Anionen und Kationen, wie Kalium, Natrium, Ammonium, Calcium, Sulfat, Carbonat, Phosphat, Chlorid, etc. Spurenelemente wie Bor, Molybdän, Kupfer, Mangan, Zink, Eisen, etc. werden in Form von Verunreinigungen der anderen Bestandteile der Medien oder als definierte Lösungen den Medien zugeführt. Die Belüftung in Flaschen und Tanks wird durchgeführt, indem man sterile Luft durch das Nährmedium hindurch leitet oder auf die Oberfläche des Nährmediums aufpreßt. Eine weitere Bewegung in den Tanks wird durch einen mechanischen Rührer gewährleistet. Falls erforderlich, kann ein Antischaum-Mittel, wie Siliconöl, zugesetzt werden.

Beispiel a

Inokulum-Herstellung

Geeignete Medien, wie sie zur Anzucht des Inokulums verwendet werden, sind Malz-Extrakt (2 %) oder Zuckerrüben-Melasse (2 %).

aa) Für die Fermentation in kleinem Maßstab

Die Nährmedien werden sterilisiert. Der gleichmäßig mit der keimfreien Kultur des Pilzes DSM 3650 bewachsene Agar (2 % Malz-Extrakt) einer Glasschale (9 cm ∅) wird in einem sterilisierten Mixer unter Zusatz von 200 ml sterilisiertem Wasser (entionisiert) homogenisiert und anschließend werden jeweils 40 ml dieses Homogenisates als Inokulum für die Kultur des Pilzes in Glaskolben (jeweils 500 ml Nährmedium) verwendet.

ab) Für die Fermentation in größerem Maßstab

Die Nährmedien werden sterilisiert. Der gleichmäßig mit der keimfreien Kultur des Pilzes DSM 3650 bewachsene Agar (2 % Malz-Extrakt) von 5 Glasschalen (9 cm ∅) wird in einem sterilisierten Mixer unter Zusatz von 400 ml sterilisiertem Waser (entionisiert) homogenisiert und dann jeweils die Hälfte dieses Homogenisates als Inokulum in 1 l Glaskolben (mit jeweils 500 ml Nährmedium) überführt. Die Medien werden anschließend 24-30 h bei 25°C auf einem Rotationsschüttler (130 bis 150 U/min) geschüttelt. Dieses Inokulum wird anschließend verwendet, um 15 l eines sterilen Zuckerrüben-Melasse-Mediums (2 %) zu beimpfen.

Beispiel b

Fermentation in kleinem Maßstab

Zur Bereitung der Fermentationsmedien wurde Zuckerrüben-Melasse (2 %) verwendet. Nach dem Beimpfen der sterilisierten Medien (jeweils 500 ml Nährmedium pro Kolben) mit dem nach Beispiel aa hergestellten Inokulum werden diese anschließend 5 bis 6 Tage bei 25°C auf einem Rotationsschüttler (130 bis 150 U/min) geschüttelt und dann die Maische geerntet.

Beispiel c

Fermentation in größerem Maßstab

Zur Bereitung der Fermentationsmedien wurde Zuckerrüben-Melasse (2 %) verwendet. Nach dem Beimpfen des sterilisierten Mediums (15 l Nährmedium) mit dem nach Beispiel ab hergestellten Inokulum wird die Fermentation bei 25°C, wobei ein steriler Luftstrom von 1,5 bis 2 l/min aufrechterhalten und unter Rühren mit einem Blattrührer, der mit 150 U/min betrieben wird, durchgeführt. Die Fermentation wird 5 bis 8 Tage betrieben und anschließend die Maische geerntet. Analog diesem Beispiel werden Fermentationen im 50 l- bzw. 100 l-Maßstab durchgeführt, wobei die Menge an Inokulum und der sterile Luftstrom den geänderten Volumina an Nährmedium entsprechend angepaßt werden.

Beispiel d

Isolierung von Verbindung Nr. 169

Ein Gesamtvolumen von ca. 80 Liter Maische, hergestellt gemäß Beispiel b oder c, wird filtriert und der Mycelkuchen verworfen. Das so erhaltene Kulturfiltrat wird in einem kontinuierlichen Extraktionsverfahren mit Dichlormethan extrahiert und aus diesem Extrakt anschließend am Rotationsverdampfer unter Vakuum das Lösungsmittel entfernt. Der Rückstand wird in einem geringen Volumen einer Mischung aus Dichlormethan : Dioxan = 95 : 5 aufgenommen und auf eine vorher mit der mobilen Phase equilibrierten Säule aufgetragen und dann druckunterstützt chromatographiert.

| Bedingungen: | | |
|---|---|---|
| Säule: | innerer Durchmesser | 5,4 cm |
| | Füllhöhe | 60,0 cm |
| stationäre Phase: | Siliciumdioxid (MN Polygosil 60) | 25–40 µ |
| mobile Phase: | Dichlormethan: Dioxan = 95 : 5 | |
| Flussrate: | 60 ml/min | |
| Druck: | 1 bar | |
| Detektion des Eluates mittels Photometer: λ = 245 nm | | |

Die den Hauptanteil an der Verbindung Nr. 169 enthaltende Fraktion wird am Rotationsverdampfer unter Vakuum zur Trockene eingeengt, dann in wenig Di-isopropylether aufgenommen und auf eine Chromatographiesäule aufgetragen.

| Bedingungen: | | |
|---|---|---|
| Säule: | innerer Durchmesser | 2,0 cm |
| | Füllhöhe | 15,0 cm |
| stationäre Phase: | Siliciumdioxid (MN Polygosil 60) | 25–40 µ |
| mobile Phase: | Di-isopropylether | |

Die den Hauptanteil an Verbindung Nr. 169 enthaltende Fraktion wird am Rotationsverdampfer unter Vakuum zur Trockne eingeengt und dann in 20 bis 50 ml Di-isopropylether aufgenommen. Man gibt das gleiche Volumen an iso-Oktan hinzu und nach einiger Zeit kristallisiert die Verbindung Nr. 169 aus. Durch nochmaliges Umkristallisieren erhält man ein sehr reines Produkt.

Herstellungsbeispiele

1. Herstellung von 2,3-Epoxy-2-benzyl-5-cyclohexen-1,4-dion

a) 19,9 g (0,1 mol) 2-Benzylbenzochinon wird in 65 ml Ethanol suspendiert und bei 0 bis 10°C unter Rühren mit 10 ml (0,11 mol) Cyclopentadien versetzt. Man läßt auf Raumtemperatur (20°C) aufwärmen, rührt 1

Std. nach und zieht das Lösungsmittel am Rotationsverdampfer ab. Umkristallisation aus Ethanol liefert 21,2 g (80 %) des Adduktes vom Typ III (R = Benzyl). Fp.: 77 - 78°C

b) Zu 19,8 g (0,075 mol) des Adduktes III in 470 ml Tetrahydrofuran (THF) wird unter Eiskühlung eine eiskalte Lösung von 17,4 g Kaliumcarbonat in 200 ml Wasser und 62,2 ml 30 % Wasserstoffperoxid zugetropft. Anschließend wird auf Raumtemperatur aufgewärmt und weitere 30 min nachgerührt. Es wird Wasser und Methylenchlorid zugesetzt, die wäßrige Phase zweimal mit Methylenchlorid extrahiert, die vereinigten organischen Phasen mit Wasser gewaschen und getrocknet. Nach Entfernen des Lösungsmittels wird das Rohprodukt aus Diethylether umkristallisiert.
Ausbeute: 20,0 g (95 %) vom Typ IV. Fp.: 98 - 99°C

c) 6 g (0,021 mol) der Verbindung vom Typ IV werden in einem Metallbad vorgeschmolzen, anschließend verdampft und der Dampf bei ca. 250 bis 270°C/0,01 Torr in einem mit Raschigringen gefüllten auf 420°C vorgeheizten Quarzrohr pyrolysiert. In einer mit Eis/Wasser gekühlten Kühlfalle wird das Rohprodukt kondensiert, in einer zweiten nachgeschalteten, mit Trockeneis gekühlten Kühlfalle wird das Cyclopentadien aufgefangen. Säulenchromatographie (Laufmittel Essigester/Pentan 1 : 9) liefert 4,3 g (94 %) gelbes Öl (Beispiel Nr. 49), IR 1691 (C=O).

2. Herstellung von 2,3-Epoxy-2-benzyl-5-cyclohexen-4-ol-1-on

Zu 2,5 g (0,0117 mol) der Verbindung Bsp. Nr. 49 in 50 ml Methanol und 5 ml Eisessig wird unter Kontrolle durch Dünnschichtchromatographie so lange portionsweise Natriumcyanoborhydrid zugesetzt, bis gerade kein Ausgangsprodukt mehr nachweisbar ist. Während der Reaktion wird die Apparatur mit Stickstoff gespült. Die Hauptmenge Methanol wird abgezogen, der Rückstand auf Wasser gegeben und mit Methylenchlorid extrahiert. Die organische Phase wird mit Wasser gewaschen und getrocknet. Nach Entfernen des Lösungsmittels wird das Rohprodukt schnell über Kieselgel filtriert (Laufmittel Essigester/Pentan 1 : 9). Ausbeute: 2,25 g (89 %) gelbliches Öl (Beispiel Nr. 189), IR: 3410 (OH), 1675 (C=O).

Die folgenden Verbindungen können in entsprechender Weise hergestellt werden.

Tabelle 1

| Bsp. | | | Physik. Daten | | |
|---|---|---|---|---|---|
| Nr. | W | R | Fp. (°C) | IR ($cm^{-1}$) | 1 H-NMR (ppm) |
| 2 | C=0 | $CH_2CH_3$ | | | |
| 3 | C=0 | $CH_2CH_2CH_3$ | | | |
| 4 | C=0 | $CH(CH_3)_2$ | | | |
| 5 | C=0 | $Ch_2CH_2CH_2CH_3$ | | | 3,90 (s, 1H, C–H) |
| 6 | C=0 | $CH^2CH(CH_3)_2$ | | | |
| 7 | C=0 | $CH(CH_3)CH_2CH_3$ | | | |
| 8 | C=0 | $C(CH_3)_3$ | 71–73 | | |
| 9 | C=0 | $CH_2CH_2CH_2CH_2CH_3$ | | | |
| 10 | C=0 | $CH_2CH_2CH(CH_3)_2$ | | | |
| 11 | C=0 | $CH_2C(CH_3)_3$ | | | |
| 12 | C=0 | $CH_2CH_2CH_2CH_2CH_2CH_3$ | | | |
| 13 | C=0 | $CH_2CH{=}CH_2$ | | | |
| 14 | C=0 | Propargyl | | | |
| 15 | C=0 | $CH_2CH{=}CH{-}CH_3$ | | | |
| 16 | C=0 | $CH_2{-}C(CH_3){=}CH_2$ | | | |
| 17 | C=0 | $CH_2{-}CH_2{-}CH{=}CH_2$ | | | |
| 18 | C=0 | 2-Butinyl | | | |
| 20 | C=0 | 3-Methyl-3-buten-1-inyl | | 1700 (C=0) | |
| 21 | C=0 | 3-Methyl-3-penten-1-inyl | | | |
| 22 | C=0 | $CH_2{-}CHBr{-}CH_2BR$ | | | |
| 23 | C=0 | $CH_2{-}CH_2{-}CCl(CH_3)_2$ | | | |
| 24 | C=0 | $CH_2{-}CH_2{-}CBr(CH_3)_2$ | | | |
| 25 | C=0 | $CH_2{-}CHBr{-}CBr(CH_3)_2$ | | | |
| 26 | C=0 | 3-Trifluormethyl-3-buten-1-inyl | | | |

Tabelle 2

R = $-(CHR^2)_m-(X)_n-(CHR^3)_p-$ 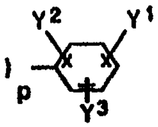

| Bsp. Nr. | W | $R^2$ | $R^3$ | X | m | n | p | $Y^1Y^2Y^3$ | Fp. (°C) | IR ($cm^{-1}$) | 1H-NMR (ppm) |
|---|---|---|---|---|---|---|---|---|---|---|---|
| 27 | C=O | - | - | - | 0 | 0 | 0 | H | | | |
| 28 | C=O | - | - | - | 0 | 0 | 0 | 3-F | | | |
| 29 | C=O | - | - | - | 0 | 0 | 0 | 4-F | | | |
| 30 | C=O | - | - | - | 0 | 0 | 0 | 2-Cl | 85 - 87 | | |
| 31 | C=O | - | - | - | 0 | 0 | 0 | 3-Cl | | | |
| 32 | C=O | - | - | - | 0 | 0 | 0 | 4-Cl | | | |
| 33 | C=O | - | - | - | 0 | 0 | 0 | 4-Br | | | |
| 34 | C=O | - | - | - | 0 | 0 | 0 | 3-$CH_3$ | | | |
| 35 | C=O | - | - | - | 0 | 0 | 0 | 4-$CH_3$ | | | |
| 36 | C=O | - | - | - | 0 | 0 | 0 | 4-t-$C_4H_9$ | | | |
| 37 | C=O | - | - | - | 0 | 0 | 0 | 3-$CF_3$ | | | |
| 38 | C=O | - | - | - | 0 | 0 | 0 | 4-$CF_3$ | | | |
| 39 | C=O | - | - | - | 0 | 0 | 0 | 3-$OCH_3$ | | | |
| 40 | C=O | - | - | - | 0 | 0 | 0 | 4-$OCH_3$ | | | |
| 41 | C=O | - | - | - | 0 | 0 | 0 | 4-$SCH_3$ | | | |
| 42 | C=O | - | - | - | 0 | 0 | 0 | 4-$NO_2$ | | | |
| 43 | C=O | - | - | - | 0 | 0 | 0 | 4-CN | | | |
| 44 | C=O | - | - | - | 0 | 0 | 0 | 4-Ph | | | |
| 45 | C=O | - | - | - | 0 | 0 | 0 | 4-OPh | | | |

Tabelle 2 - Forts.

| Bsp. Nr. | W | $R^2$ | $R^3$ | X | m | n | p | $Y^1Y^2Y^3$ | Physik. Daten Fp. ($^0C$) | IR ($cm^{-1}$) | 1H-NMR (ppm) |
|---|---|---|---|---|---|---|---|---|---|---|---|
| 46 | C=O | - | - | - | 0 | 0 | 0 | 2,4-$Cl_2$ | 119 - 120 | | |
| 47 | C=O | - | - | - | 0 | 0 | 0 | 2,4-$Br_2$ | | | |
| 48 | C=O | - | - | - | 0 | 0 | 0 | 2,4,5-$Cl_3$ | | | |
| 49 | C=O | H | - | - | 1 | 0 | 0 | H | | 1691 (C=O) | |
| 50 | C=O | H | - | - | 1 | 0 | 0 | 3-F | | | |
| 51 | C=O | H | - | - | 1 | 0 | 0 | 4-F | | | 3,30 (s) 2H; 3,65 (d) 1H; 6,74 (m) 2H; |
| 52 | C=O | H | - | - | 1 | 0 | 0 | 3-Cl | | | |
| 53 | C=O | H | - | - | 1 | 0 | 0 | 4-Cl | | | |
| 54 | C=O | H | - | - | 1 | 0 | 0 | 4-t-$C_4H_9$ | | | |
| 55 | C=O | H | - | - | 1 | 0 | 0 | 3-$CF_3$ | | | |
| 56 | C=O | H | - | - | 1 | 0 | 0 | 4-$CF_3$ | | | |
| 57 | C=O | H | - | - | 1 | 0 | 0 | 3-$OCH_3$ | | | |
| 58 | C=O | H | - | - | 1 | 0 | 0 | 4-$OCH_3$ | | | |
| 59 | C=O | H | - | - | 1 | 0 | 0 | 3-CN | | | |
| 60 | C=O | H | - | - | 1 | 0 | 0 | 4-CN | | | |
| 61 | C=O | H | - | - | 1 | 0 | 0 | 3-NO | | | |
| 62 | C=O | H | - | - | 1 | 0 | 0 | 4-$NO_2$ | | | |
| 63 | C=O | $CH_3$ | - | - | 1 | 0 | 0 | H | | | |
| 64 | C=O | $CH_3$ | - | - | 1 | 0 | 0 | 2-Cl | | | |
| 65 | C=O | $CH_3$ | - | - | 1 | 0 | 0 | 4-Cl | | | |
| 66 | C=O | $CH_3$ | - | - | 1 | 0 | 0 | 2-$OCH_3$ | | | |
| 67 | C=O | $CH_3$ | - | - | 1 | 0 | 0 | 3-$OCH_3$ | | | |
| 68 | C=O | $CH_3$ | - | - | 1 | 0 | 0 | 4-$OCH_3$ | | | |
| 69 | C=O | $CH_3$ | - | - | 1 | 0 | 0 | 2-$NO_2$ | | | |
| 70 | C=O | $CH_3$ | - | - | 1 | 0 | 0 | 4-$NO_2$ | | | |

Tabelle 2 - Forts.

| Bsp. Nr. | W | $R^2$ | $R^3$ | X | m | n | p | $Y^1Y^2Y^3$ | Physik. Daten Fp. (°C) | IR ($cm^{-1}$) | 1H-NMR (ppm) |
|---|---|---|---|---|---|---|---|---|---|---|---|
| 71 | C=O | H | - | 0 | 1 | 1 | 0 | H | 125 - 125,5 | | |
| 72 | C=O | H | - | 0 | 1 | 1 | 0 | 3-F | | | |
| 73 | C=O | H | - | 0 | 1 | 1 | 0 | 4-F | | | |
| 74 | C=O | H | - | 0 | 1 | 1 | 0 | 2-Cl | | | |
| 75 | C=O | H | - | 0 | 1 | 1 | 0 | 3-Cl | | | |
| 76 | C=O | H | - | 0 | 1 | 1 | 0 | 4-Cl | | | |
| 77 | C=O | H | - | 0 | 1 | 1 | 0 | 4-Br | | | |
| 78 | C=O | H | - | 0 | 1 | 1 | 0 | 2,4-$Cl_2$ | 127 - 129 | | |
| 79 | C=O | H | - | 0 | 1 | 1 | 0 | 3,4-$Cl_2$ | | | |
| 80 | C=O | H | - | 0 | 1 | 1 | 0 | 2,4,5-$Cl_3$ | | | |
| 81 | C=O | H | - | 0 | 1 | 1 | 0 | 4-t-$C_4H_9$ | | | |
| 82 | C=O | H | - | 0 | 1 | 1 | 0 | 4-Ph | | | |
| 83 | C=O | H | - | 0 | 1 | 1 | 0 | 4-OPh | | | |
| 84 | C=O | H | - | 0 | 1 | 1 | 0 | 2-$OCH_3$ | 83 - 85 | | |
| 85 | C=O | H | - | 0 | 1 | 1 | 0 | 3-$OCH_3$ | | | |
| 86 | C=O | H | - | 0 | 1 | 1 | 0 | 4-$OCH_3$ | | | |
| 87 | C=O | H | - | 0 | 1 | 1 | 0 | 4-CN | | | |
| 88 | C=O | H | - | 0 | 1 | 1 | 0 | 4-CN | | | |
| 89 | C=O | H | - | 0 | 1 | 1 | 0 | 2-$NO_2$ | | | |
| 90 | C=O | H | - | 0 | 1 | 1 | 0 | 3-$NO_2$ | | | |
| 91 | C=O | H | - | 0 | 1 | 1 | 0 | 3-$CF_3$ | | | |
| 92 | C=O | H | - | 0 | 1 | 1 | 0 | 4-$CF_3$ | | | |
| 93 | C=O | H | - | 0 | 1 | 1 | 0 | 4-$SCH_3$ | | | |
| 94 | C=O | H | - | 0 | 1 | 1 | 0 | 4-$SO_2CH_3$ | | | |

Tabelle 2 - Forts.

| Bsp. Nr. | W | $R^2$ | $R^3$ | X | m | n | p | $Y^1Y^2Y^3$ | Physik. Daten Fp. (°C) | IR ($cm^{-1}$) | 1H-NMR (ppm) |
|---|---|---|---|---|---|---|---|---|---|---|---|
| 95 | C=O | $CH_3$ | - | O | 1 | 1 | 0 | H | | | |
| 96 | C=O | $CH_3$ | - | O | 1 | 1 | 0 | 4-F | | | |
| 97 | C=O | $CH_3$ | - | O | 1 | 1 | 0 | 4-Cl | | | |
| 98 | C=O | $CH_3$ | - | O | 1 | 1 | 0 | 2,4-$Cl_2$ | | | |
| 99 | C=O | $CH_3$ | - | O | 1 | 1 | 0 | 2,4,5-$Cl_3$ | | | |
| 100 | C=O | $CH_3$ | - | O | 1 | 1 | 0 | 4-t-$C_4H_9$ | | | |
| 101 | C=O | $CH_3$ | - | O | 1 | 1 | 0 | 4-$OCH_3$ | | | |
| 102 | C=O | $CH_3$ | - | O | 1 | 1 | 0 | 4-$CF_3$ | | | |
| 103 | C=O | $CH_3$ | - | O | 1 | 1 | 0 | 4-CN | | | |
| 104 | C=O | $CH_3$ | - | O | 1 | 1 | 0 | 4-$NO_2$ | | | |
| 105 | C=O | $CH_3$ | - | O | 1 | 1 | 0 | 4-O 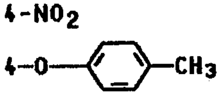 | | | |
| 106 | C=O | H | - | S | 1 | 1 | 0 | H | | | |
| 107 | C=O | H | - | S | 1 | 1 | 0 | 4-Cl | | | |
| 108 | C=O | - | H | O | 0 | 1 | 1 | H | | | |
| 109 | C=O | - | H | O | 0 | 1 | 1 | 3-F | | | |
| 110 | C=O | - | H | O | 0 | 1 | 1 | 4-F | | | |
| 111 | C=O | - | H | O | 0 | 1 | 1 | 3-Cl | | | |
| 112 | C=O | - | H | O | 0 | 1 | 1 | 4-Cl | | | |
| 113 | C=O | - | H | O | 0 | 1 | 1 | 4-Br | | | |
| 114 | C=O | - | H | O | 0 | 1 | 1 | 2-$CH_3$ | | | |
| 115 | C=O | - | H | O | 0 | 1 | 1 | 3-$CH_3$ | | | |
| 116 | C=O | - | H | O | 0 | 1 | 1 | 4-$CH_3$ | | | |

Tabelle 2 - Forts.

| Bsp. Nr. | W | $R^2$ | $R^3$ | X | m | n | p | $Y^1Y^2Y^3$ | Physik. Daten Fp. (°C) | IR ($cm^{-1}$) | 1H-NMR (ppm) |
|---|---|---|---|---|---|---|---|---|---|---|---|
| 117 | C=O | - | H | O | 0 | 1 | 1 | 4-t-$C_4H_9$ | | | |
| 118 | C=O | - | H | O | 0 | 1 | 1 | 4-$CF_3$ | | | |
| 119 | C=O | - | H | O | 0 | 1 | 1 | 2,4-$Cl_2$ | | | |
| 120 | C=O | - | H | O | 0 | 1 | 1 | 3-CN | | | |
| 121 | C=O | - | H | O | 0 | 1 | 1 | 4-CN | | | |
| 122 | C=O | - | H | O | 0 | 1 | 1 | 4-$NO_2$ | | | |
| 123 | C=O | - | $CH_3$ | O | 0 | 1 | 1 | H | | | |
| 124 | C=O | - | $CH_3$ | O | 0 | 1 | 1 | 2,5-$(CH_3)_2$ | | | |
| 125 | C=O | H | H | O | 3 | 1 | 1 | H | | | |
| 126 | C=O | H | H | O | 3 | 1 | 1 | 4-F | | | |
| 127 | C=O | H | H | O | 3 | 1 | 1 | 4-Cl | | | |
| 128 | C=O | H | H | O | 3 | 1 | 1 | 4-$CH_3$ | | | |
| 129 | C=O | H | H | O | 3 | 1 | 1 | 4-$CF_3$ | | | |
| 130 | C=O | H | H | O | 3 | 1 | 1 | 4-CN | | | |
| 131 | C=O | H | H | O | 3 | 1 | 1 | 4-$NO_2$ | | | |
| 132 | C=O | H | $CH_3$ | O | 3 | 1 | 1 | H | | | |
| 133 | C=O | H | $CH_3$ | O | 3 | 1 | 1 | 2,5-$(CH_3)_2$ | | | |
| 134 | C=O | H | - | O | 3 | 1 | 0 | H | | | |
| 135 | C=O | H | - | O | 3 | 1 | 0 | 4-F | | | |
| 136 | C=O | H | - | O | 3 | 1 | 0 | 4-Cl | | | |
| 137 | C=O | H | - | O | 3 | 1 | 0 | 3-$CH_3$ | | | |
| 138 | C=O | H | - | O | 3 | 1 | 0 | 4-$CH_3$ | | | |
| 139 | C=O | H | - | O | 3 | 1 | 0 | 4-$CF_3$ | | | |
| 140 | C=O | H | - | S | 3 | 1 | 0 | H | | | |

Tabelle 2 - Forts.

| Bsp. Nr. | W | $R^2$ | $R^3$ | X | m | n | p | $Y^1Y^2Y^3$ | Physik. Daten Fp. ($^0C$) | IR ($cm^{-1}$) | 1H-NMR (ppm) |
|---|---|---|---|---|---|---|---|---|---|---|---|
| 141 | C=O | H | - | S | 3 | 1 | 0 | 4-F | | | |
| 142 | C=O | H | - | S | 3 | 1 | 0 | 4-Cl | | | |
| 143 | C=O | H | - | S | 3 | 1 | 0 | $3-CH_3$ | | | |
| 144 | C=O | H | - | S | 3 | 1 | 0 | $4-CH_3$ | | | |
| 145 | C=O | H | - | S | 3 | 1 | 0 | $4-CF_3$ | | | |

Tabelle 3

| Bsp. Nr. | W | $R^1$ | R | Physik. Daten Fp. (°C) | IR ($cm^{-1}$) | 1H-NMR (ppm) |
|---|---|---|---|---|---|---|
| 146 | $CHOR^1$ | H | $CH_3$ | | | |
| 147 | $CHOR^1$ | $COCH_3$ | $CH_3$ | | | |
| 148 | $CHOR^1$ | $CH_3$ | $CH_3$ | | | |
| 149 | $CHOR^1$ | $CH_2-CH=CH_2$ | $CH_3$ | | | |
| 150 | $CHOR^1$ | H | $n-CH_2-CH_2CH_3$ | | | |
| 151 | $CHOR^1$ | $COCH_3$ | $n-CH_2-CH_2CH_3$ | | | |
| 152 | $CHOR^1$ | $CH_3$ | $n-CH_2-CH_2CH_3$ | | | |
| 153 | $CHOR^1$ | H | $CH(CH_3)_2$ | | | |
| 154 | $CHOR^1$ | $COCH_3$ | $CH(CH_3)_2$ | | | |
| 155 | $CHOR^1$ | $CH_3$ | $CH(CH_3)_2$ | | | |
| 156 | $CHOR^1$ | H | $CH_2CH_2CH_2CH_3$ | | 1685 (C=O)<br>3415 (OH) | |
| 157 | $CHOR^1$ | $COCH_3$ | $CH_2CH_2CH_2CH_3$ | | | |
| 158 | $CHOR^1$ | $CH_3$ | $CH_2CH_2CH_2CH_3$ | | | |
| 159 | $CHOR^1$ | $CH(CH_3)_2$ | $CH_2CH_2CH_2CH_3$ | | | |
| 160 | $CHOR^1$ | $CH_2-CH=CH_2$ | $CH_2CH_2CH_2CH_3$ | | | |
| 161 | $CHOR^1$ | H | $C(CH_3)_3$ | | | 3,81 (m,1H,C-H)<br>4,62 (m,1H,C-H) |
| 162 | $CHOR^1$ | $COCH_3$ | $C(CH_3)_3$ | | | |
| 163 | $CHOR^1$ | H | $CH_2CH=CHCH_3$ | | | |
| 164 | $CHOR^1$ | $COCH_3$ | $CH_2CH=CHCH_3$ | | | |
| 165 | $CHOR^1$ | $CH_3$ | $CH_2CH=CHCH_3$ | | | |
| 166 | $CHOR^1$ | H | $CH_2-CH=C(CH_3)_2$ | | | 3,64 (m,1H,C-H)<br>5,04 (m,1H,C-H) |
| 167 | $CHOR^1$ | $COCH_3$ | $CH_2-CH=C(CH_3)_2$ | | | |
| 168 | $CHOR^1$ | $CH_3$ | $CH_2-CH=C(CH_3)_2$ | | | |
| 169 | $CHOR^1$ | H | $-C\equiv C-C=CH_2$<br>\|<br>$CH_3$ | | 1664, 2217<br>3450 | 1,0 (3H,s); 4,05 (1H,s); 4,5 (1H,m); 5,4-5,46 (1H,d); 5,95-6,05 (2H,m); 6,7-6,8 (1H, m) |

Tabelle 4

R = $-(CHR^2)_m-(X)_n-(CHR^3)_p-$ 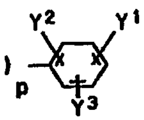

| Bsp. Nr. | W | $R^1$ | $R^2$ | $R^3$ | X | m | n | p | $Y^1Y^2Y^3$ | Fp. (°C) | Physik. Daten IR ($cm^{-1}$) | 1H-NMR (ppm) |
|---|---|---|---|---|---|---|---|---|---|---|---|---|
| 170 | $CHOR^1$ | H | - | - | - | 0 | 0 | 0 | H | | | |
| 171 | $CHOR^1$ | $COCH_3$ | - | - | - | 0 | 0 | 0 | H | | | |
| 172 | $CHOR^1$ | $CH_3$ | - | - | - | 0 | 0 | 0 | H | | | |
| 173 | $CHOR^1$ | H | - | - | - | 0 | 0 | 0 | 4-F | | | |
| 174 | $CHOR^1$ | $COCH_3$ | - | - | - | 0 | 0 | 0 | 4-F | | | |
| 175 | $CHOR^1$ | H | - | - | - | 0 | 0 | 0 | 2-Cl | | | |
| 176 | $CHOR^1$ | $COCH_3$ | - | - | - | 0 | 0 | 0 | 2-Cl | | | |
| 177 | $CHOR^1$ | $CH_3$ | - | - | - | 0 | 0 | 0 | 2-Cl | | | |
| 178 | $CHOR^1$ | H | - | - | - | 0 | 0 | 0 | 4-Cl | | | |
| 179 | $CHOR^1$ | H | - | - | - | 0 | 0 | 0 | $3-CH_3$ | | | |
| 180 | $CHOR^1$ | H | - | - | - | 0 | 0 | 0 | $4-CH_3$ | | | |
| 181 | $CHOR^1$ | H | - | - | - | 0 | 0 | 0 | $4-t-C_4H_9$ | | | |
| 182 | $CHOR^1$ | H | - | - | - | 0 | 0 | 0 | $3-OCH_3$ | | | |
| 183 | $CHOR^1$ | H | - | - | - | 0 | 0 | 0 | $4-OCH_3$ | | | |
| 184 | $CHOR^1$ | H | - | - | - | 0 | 0 | 0 | 4-Ph | | | |
| 185 | $CHOR^1$ | $COCH_3$ | - | - | - | 0 | 0 | 0 | 4-Ph | | | |
| 186 | $CHOR^1$ | H | - | - | - | 0 | 0 | 0 | $2,4-Cl_2$ | | 3417 (OH)<br>1671 (C=O) | |
| 187 | $CHOR^1$ | $COCH_3$ | - | - | - | 0 | 0 | 0 | $2,4-Cl_2$ | | | |
| 188 | $CHOR^1$ | $CH_3$ | - | - | - | 0 | 0 | 0 | $2,4-Cl_2$ | | | |
| 189 | $CHOR^1$ | H | H | - | - | 1 | 0 | 0 | H | | 3410 (OH)<br>1675 (C=O) | |

Tabelle 4 - Forts.

| Bsp. Nr. | W | $R^1$ | $R^2$ | $R^3$ | X | m | n | p | $Y^1Y^2Y^3$ | Fp. ($^0C$) | Physik. Daten IR ($cm^{-1}$) | 1H-NMR (ppm) |
|---|---|---|---|---|---|---|---|---|---|---|---|---|
| 190 | $CHOR^1$ | $COCH_3$ | H | - | - | 1 | 0 | 0 | H | | | |
| 191 | $CHOR^1$ | $CH_3$ | H | - | - | 1 | 0 | 0 | H | | | |
| 192 | $CHOR^1$ | | H | - | - | 1 | 0 | 0 | H | | | |
| 193 | $CHOR^1$ | H | H | - | - | 1 | 0 | 0 | 3-F | | | |
| 194 | $CHOR^1$ | H | H | - | - | 1 | 0 | 0 | 4-F | | | |
| 195 | $CHOR^1$ | H | H | - | - | 1 | 0 | 0 | 3-Cl | | | |
| 196 | $CHOR^1$ | H | H | - | - | 1 | 0 | 0 | 4-Cl | | | |
| 197 | $CHOR^1$ | H | H | - | - | 1 | 0 | 0 | $4-t-C_4H_9$ | | | |
| 198 | $CHOR^1$ | H | H | - | - | 1 | 0 | 0 | $4-OCH_3$ | | | |
| 199 | $CHOR^1$ | H | $CH_3$ | - | - | 1 | 0 | 0 | H | | | |
| 200 | $CHOR^1$ | H | $CH_3$ | - | - | 1 | 0 | 0 | 2-Cl | | | |
| 201 | $CHOR^1$ | H | H | - | 0 | 1 | 1 | 0 | H | | 3436 (OH)<br>1686 (C=O) | |
| 202 | $CHOR^1$ | $COCH_3$ | H | - | 0 | 1 | 1 | 0 | H | | | |
| 203 | $CHOR^1$ | $CH_3$ | H | - | 0 | 1 | 1 | 0 | H | | | |
| 204 | $CHOR^1$ | H | H | - | 0 | 1 | 1 | 0 | 3-F | | | |
| 205 | $CHOR^1$ | H | H | - | 0 | 1 | 1 | 0 | 4-F | | | |
| 206 | $CHOR^1$ | H | H | - | 0 | 1 | 1 | 0 | 3-Cl | | | |
| 207 | $CHOR^1$ | H | H | - | 0 | 1 | 1 | 0 | 4-Cl | | | |
| 208 | $CHOR^1$ | H | H | - | 0 | 1 | 1 | 0 | $2,4-Cl_2$ | | | |
| 209 | $CHOR^1$ | $COCH_3$ | H | - | 0 | 1 | 1 | 0 | $2,4-Cl_2$ | | | |
| 210 | $CHOR^1$ | H | $CH_3$ | - | 0 | 1 | 1 | 0 | H | | | |
| 211 | $CHOR^1$ | H | $CH_3$ | - | 0 | 1 | 1 | 0 | $2,4-Cl_2$ | | | |
| 212 | $CHOR^1$ | H | H | - | S | 1 | 1 | 0 | H | | | |
| 213 | $CHOR^1$ | H | - | H | 0 | 0 | 1 | 1 | H | | | |

Tabelle 4 - Forts.

| Bsp. Nr. | W | $R^1$ | $R^2$ | $R^3$ | X | m | n | p | $Y^1Y^2Y^3$ | Fp. ($^0C$) | Physik. Daten IR ($cm^{-1}$) | 1H-NMR (ppm) |
|---|---|---|---|---|---|---|---|---|---|---|---|---|
| 214 | $CHOR^1$ | H | - | H | O | 0 | 1 | 1 | 4-Cl | | | |
| 215 | $CHOR^1$ | H | - | H | O | 0 | 1 | 1 | $4-CH_3$ | | | |
| 216 | $CHOR^1$ | H | - | $CH_3$ | O | 0 | 1 | 1 | H | | | |
| 217 | $CHOR^1$ | H | H | H | O | 3 | 1 | 1 | H | | | |
| 218 | $CHOR^1$ | H | H | H | O | 3 | 1 | 1 | 4-Cl | | | |
| 219 | $CHOR^1$ | H | H | H | O | 3 | 1 | 1 | $4-CH_3$ | | | |
| 220 | $CHOR^1$ | H | H | $CH_3$ | O | 3 | 1 | 1 | H | | | |
| 221 | $CHOR^1$ | H | H | - | O | 3 | 1 | 0 | H | | | |
| 222 | $CHOR^1$ | H | H | - | O | 3 | 1 | 0 | 4-Cl | | | |
| 223 | $CHOR^1$ | H | H | - | O | 3 | 1 | 0 | $3-CH_3$ | | | |
| 224 | $CHOR^1$ | H | H | - | S | 3 | 1 | 0 | H | | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Phycomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Phytophthora infestans an Tomaten und Kartoffeln,
Phytophthora parasitica an Erdbeeren,
Pseudoperonospora cubensis an Gurken,
Pseudoperonospora humuli an Hopfen,
Peronospora destructor an Zwiebeln,
Peronospora tabacina an Tabak,
Plasmopara viticola an Reben,
Plasmopara halstedii an Sonnenblumen,
Sclerospora macrospora an Mais,
Bremia lactucae an Salat,
Mucor mucedo an Früchten,
Rhizopus nigricans an Rüben sowie
Erysiphe graminis an Getreide,
Uncinula necator an Reben,
Sphaerotheca fuliginea an Rosen,
Puccinia-Arten an Getreide,
Rhizoctonia-Arten an Baumwolle,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die neuen Verbindungen können auch im Materialschutz eingesetzt werden z.B. gegen Paecilomyces variotii.

Einige der neuen Verbindungen haben sehr gute Wirksamkeiten gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 71 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 189 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 201 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 71 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 189 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 201 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 71 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 189 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 71 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,

2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitrio-5-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-Trichlormethylthio-tetrahydrophthalimid (A) und 2-Hydroxymethyl-2,3-epoxy-5-cyclohexen-1,4-dion (B) benutzt.

Anwendungsbeispiel 1

Wirksamkeit gegen Phytophthora infestans an Tomaten

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.
Das Ergebnis des Versuches zeigt, daß bei der Anwendung als 0,025 %ige (Gew.%) Spritzbrühe beispielsweise die Verbindungen Nr. 71, 189 und 201 eine bessere fungizide Wirkung zeigten (90 %) als die bekannten Vergleichswirkstoffe A (70 %) und B (10 %).

Anwendungsbeispiel 2

Wirksamkeit gegen Plasmopara viticola

Blätter von Topfreben der Sorte "Müller-Thurgau" wurden mit wäßriger Spritzbrühe, die 80 % Wirkstoff und 20 % Emulgiermittel in der Trockensubstanz enthielten, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.
Das Ergebnis des Versuches zeigt, daß z.B. der Wirkstoff Nr. 71 bei der Anwendung als 0,05 %ige Spritzbrühe eine bessere fungizide Wirkung zeigt (90 %) als der Vergleichswirkstoff B (60 %).

Anwendungsbeispiel 3

Wirksamkeit gegen Rhizoctonia solani (Gießbehandlung)

Dampf-sterilisierte Erde wurde mit Rhizoctonia solani - dem Erreger der Stengelfäule - inoculiert durch Zumischen eines Maismehl-Quarzsand-Gemisches, das völlig mit Rhizoctonia solani-Mycel durchwachsen war. Pro kg Erde wurde 1 g dieses Gemisches zugesetzt. Pro Schale wurden 20 Baumwollsamen der Sorte "Stoneville" in diese Erde ausgesät. 6 Stunden später wurde die Schale mit 50 ml einer wäßrigen Wirkstoffsuspension gegossen und anschließend für 3 Wochen im Gewächshaus kultiviert. Dann wurde der Wurzelansatz aller Pflanzen auf Symptome untersucht.

Auswertung: Angabe der Anzahl gesunder Pflanzen.

Als Vergleichsmittel diente die aus US 3 541 213 bekannte Verbindung 1-(N-Butylcarbamoyl)-2-(methoxy-carboxamido)-benzimidazol (C).

Tabelle

| Wirkstoff Nr. | Anzahl gesunder Baumwollpflanzen nach Giessbehandlung mit ...%iger wässriger Wirkstoffaufbereitung | |
|---|---|---|
| | 0,05 | 0,0125 |
| C | 15 | 2 |
| 169 | 17 | 17 |
| Unbehandelte Kontrolle | 0 | 0 |

Die starke fungizide Wirkung ist deutlich erkennbar.

Anwendungsbeispiel 4

Die Wirkung der Verbindung Nr. 169 auf das Wachstum von erwünschten und unerwünschten Beispielspflanzen wird anhand von Gewächshausversuchen vorgeführt:

Als Kulturgefäße dienen Pflastikblumentöpfe mit 300 $cm^3$ Inhalt und lehmigem Sand mit etwa 3 % Humus als Substrat. Die Samen der Testpflanzen wurde nach Arten getrennt flach eingesät. Bestimmte Arten werden erst als Keimpflanzen in Saatschalen getrennt angezogen und einige Tage vor der Behandlung in die Versuchsgefäße verpflanzt. Die Wirkstoffe - hier Verbindung Nr. 169 - werden in einem Lösungsmittel gelöst und in Wasser als Verteilungsmittel emulgiert und mittels fein verteilender Düsen gespritzt. Die Aufwandmenge beträgt 2,0 kg Wirkstoff/ha.

Bei den Beispielspflanzen handelt es sich um:

| Botanischer Name | Deutscher Name | Englischer Name |
|---|---|---|
| Arachis hypogaea | Erdnuss | peanuts (groundnuts) |
| Chenopodium album | Weisser Gänsefuss | lambsquaters (goosefoot) |
| Euphorbia heterophylla | Wolfsmilchart | spurge |
| Medicago sativa | Luzerne | alfalfa |
| Mercurialis annua | einj. Bingelkraut | annual mercury |
| Nicotiana rustica | Tabak | tabacco |
| Sinapis arvensis | Ackersenf | yellow charlock |

Die Versuchsgefäße werden im Gewächshaus aufgestellt, wobei für wärmeliebende Arten wärme Bereiche (20 bis 35°C) und für solche gemäßigter Klimate 10 bis 20°C bevorzugt werden. Die Versuchsperiode erstreckt sich bis zu 4 Wochen. Während dieser Zeit werden die Pflanzen gepflegt und ihre Reaktion auf die einzelnen Behandlungen ausgewertet. Bewertet wird nach einer Skala von 0 bis 100. Dabei bedeutet 0 keine Schädigung oder normale Wachstum und 100 völlige Zerstörung zumindest der oberirdischen Teile.

Selektive herbizide Wirkung von Verbindung Nr. 169 bei Nachauflaufanwendung im Gewächshaus

| Testpflanzen | Schädigung % bei 2,0 kg Wirkstoff/ha |
|---|---|
| Arachys hypogaea | 10 |
| Medicago sativa | 0 |
| Nicotiana rustica | 0 |
| Chenopodium album | 100 |
| Euphorbia heterophylla | 100 |
| Mercuralis annua | 95 |
| Sinapis arvensis | 93 |

Die Verbindung Nr. 169 bekämpft mit 2,0 kg Wirkstoff/ha unerwünschte breitblättrige Pflanzen. Gewisse breitblättrige Kulturpflanzen erleiden dabei keine oder nur geringe und akzeptable temporäre Schädigungen.

Sind gewisse Kulturpflanzen gegenüber dem Mittel etwa empfindlicher, so können auch Ausbringungstechniken angewandt werden, bei welchen das herbizide Mittel mit Hilfe der Spritzgeräte so geleitet wird, daß die Blätter empfindlicher Kulturpflanzen nach Möglichkeit nicht getroffen werden, während es auf die Blätter darunter wachsender unerwünschter Pflanzen oder die unbedeckte Bodenfläche gelangt (post-directed, lay-by). In Anbetracht der Verträglichkeit und der Vielseitigkeit der Applikationsmethoden und auch angesichts der fungiziden Wirkung der Verbindung Nr. 169 kann diese in einer großen Zahl von Kulturpflanzen zu deren Schutz eingesetzt werden. Die Aufwandmengen können dabei zwischen 0,02 und 10,0 kg/h und mehr, insbesondere 0,05 und 3 kg/ha schwanken.

Es ist ferner nützlich, die neue Verbindung in Kombination mit anderen Herbiziden, anderen Fungiziden und weiteren Pflanzenbehandlungsmitteln gemischt, gemeinsam auszubringen. Von interesse ist ferner die Mischbarkeit mit Mineralsalzlösungen, welche zur Behebung von Ernährungs- und Spurenelementmängeln eingesetzt werden. Ferner dienen Tenside und nicht phytotoxische Öle bzw. Ölkonzentrate zur Wirkungsverbesserung.

**Patentansprüche**

1. 2-substituierte 2,3-Epoxy-5-cyclohexenonderivate der Formel I

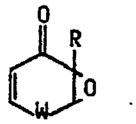

(I)

in welcher
W C=O oder $CHOR^1$,
$R^1$ Wasserstoff, $C_1$-$C_4$-Alkyl, $C_2$-$C_4$-Alkenyl,
$C_2$-$C_4$-Alkinyl oder $C_2$-$C_4$-Alkylcarbonyl
R eine gesättigte oder ungesättigte Kohlenwasserstoffkette mit 1 bis 10 C-Atomen, die gegebenenfalls durch Halogen substituiert ist, oder den Rest

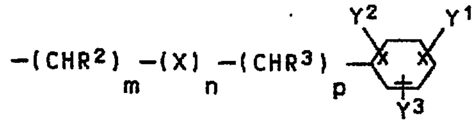

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl,
$C_1$-$C_4$-Alkylthio, $C_1$-$C_4$-Halogenalkoxy, $C_1$-$C_4$-Halogenalkylthio,
$C_1$-$C_4$-Alkylsulfonyl, Halogen, Cyano, Nitro, gegebenenfalls substituiertes Phenyl oder gegebenenfalls substituiertes Phenoxy
m, p unabhängig voneinander 0, 1, 2 oder 3,
n 0 oder 1 bedeuten, außer den Verbindungen in denen W den Rest C=0 und R Methyl oder 3-Methyl-2-butenyl bedeutet.

2. 2-Substituierte 2,3-Epoxy-5-cyclohexenonderivate der Formel I gemäß Anspruch 1, dadurch gekennzeichnet, daß
W C=O oder $CHOR^1$,
$R^1$ Wasserstoff,

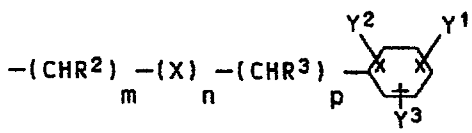

$R^2$, $R^3$ unabhängig voneinander Wasserstoff oder Methyl,
X Sauerstoff oder Schwefel,
$Y^1$, $Y^2$, $Y^3$ Wasserstoff, $C_1$-$C_4$-Alkyl, $C_1$-$C_4$-Alkoxy, $C_1$-$C_4$-Halogenalkyl, Halogen oder Nitro,
m, n und p unabhängig voneinander 0 oder 1 bedeuten.

3. 2,3-Epoxy-2-phenoxymethyl-5-cyclohexen-1,4-dion.

4. 2,3-Epoxy-2(3-methyl-3-buten-1-inyl)-5-cyclohexen-4-ol-1-on.

5. Fungizid, enthaltend einen inerten Zusatzstoff und ein 2-substituiertes 2,3-Epoxy-5-cyclohexenonderivat I der Formel I

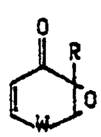

(I)

in welcher
W C=0 oder $CHOR^1$,
$R^1$ Wasserstoff, $C_1$-$C_4$-Alkyl, $C_2$-$C_4$-Alkenyl,
$C_2$-$C_4$-Alkinyl oder $C_2$-$C_4$-Alkylcarbonyl
R eine gesättigte oder ungesättigte Kohlenwasserstoffkette mit 1 bis 10 C-Atomen, die gegebenenfalls durch Halogen substituiert ist, oder den Rest

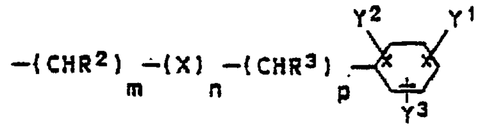

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl, $C_1$-$C_4$-Alkyithio, $C_1$-$C_4$-Halogenalkoxy, $C_1$-$C_4$-Halogenalkylthio, $C_1$-$C_4$-Alkylsulfonyl, Halogen, Cyano, Nitro, gegenbenenfalls substituiertes Phenyl oder gegebenenfalls substituiertes Phenoxy
m, p unabhängig voneinander 0, 1, 2 oder 3,
n 0 oder 1 bedeuten.

6. Verfahren zur Bekämpfung von Pilzen, dadurch gekennzeichnet, daß man diese oder die vor Pilzbefall zu schützenden Saatgüter, Pflanzen, Materialien oder den Boden mit einem 2-substituierten 2,3-Epoxy-5-cyclohexenonderivat I der Formel I

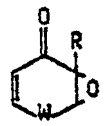
(I)

in welcher
W C=0 oder $CHOR^1$,
$R^1$ Wasserstoff, $C_1$-$C_4$-Alkyl, $C_2$-$C_4$-Alkenyl, $C_2$-$C_4$-Alkinyl oder $C_2$-$C_4$-Alkylcarbonyl
R eine gesättigte oder ungesättigte Kohlenwasserstoffkette mit 1 bis 10 C-Atomen, die gegebenenfalls durch Halogen substituiert ist, oder den Rest

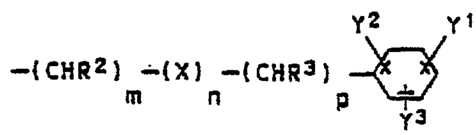

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl, $C_1$-$C_4$-Alkyithio, $C_1$-$C_4$-Halogenalkoxy, $C_1$-$C_4$-Halogenalkylthio, $C_1$-$C_4$-Alkylsulfonyl, Halogen, Cyano, Nitro, gegenbenenfalls substituiertes Phenyl oder gegebenenfalls substituiertes Phenoxy
m, p unabhängig voneinander 0, 1, 2 oder 3,
n 0 oder 1 bedeuten,
behandelt.

7. Herbizid, enthaltend einen inerten Zusatzstoff und ein 2-substituiertes 2,3-Epoxy-5-cyclohexenonderivat der Formel I

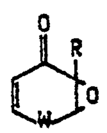
(I)

in welcher
W C=0 oder $CHOR^1$,
$R^1$ Wasserstoff, $C_1$-$C_4$-Alkyl, $C_2$-$C_4$-Alkenyl, $C_2$-$C_4$-Alkinyl oder $C_2$-$C_4$-Alkylcarbonyl
R eine gesättigte oder ungesättigte Kohlenwasserstoffkette mit 1 bis 10 C-Atomen, die gegebenenfalls durch Halogen substituiert ist, oder den Rest

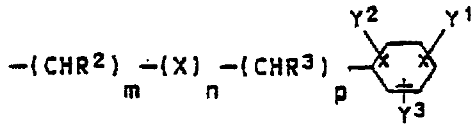

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl, $C_1$-$C_4$-Alkyithio, $C_1$-$C_4$-Halogenalkoxy, $C_1$-$C_4$-Halogenalkylthio, $C_1$-$C_4$-Alkylsulfonyl, Halogen, Cyano, Nitro, gegenbenenfalls substituiertes Phenyl oder gegebenenfalls substituiertes Phenoxy
m, p unabhängig voneinander 0, 1, 2 oder 3,
n 0 oder 1 bedeuten.

8. Verfahren zur Bekämpfung von unerwünschtem Pflanzenwuchs, dadurch gekennzeichnet, daß man die Pflanzen oder den von unerwünschtem Pflanzenwuchs freizuhaltenden Boden mit einem 2-substituierten 2,3-Epoxy-5-cyclohexenonderivat I der Formel I

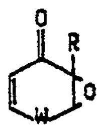

(I)

in welcher
W C=0 oder $CHOR^1$,
$R^1$ Wasserstoff, $C_1$-$C_4$-Alkyl, $C_2$-$C_4$-Alkenyl, $C_2$-$C_4$-Alkinyl oder $C_2$-$C_4$-Alkylcarbonyl
R eine gesättigte oder ungesättigte Kohlenwasserstoffkette mit 1 bis 10 C-Atomen, die gegebenenfalls durch Halogen substituiert ist, oder den Rest

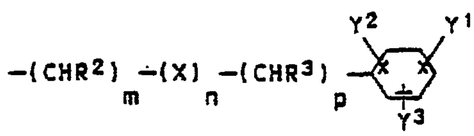

$R^2$, $R^3$ unabhängig voneinander Wasserstoff oder Methyl
X Sauerstoff oder Schwefel
$Y^1$, $Y^2$, $Y^3$ Wasserstoff, $C_1$-$C_4$-Alkyl, $C_1$-$C_4$-Alkoxy, $C_1$-$C_4$-Halogenalkyl, $C_1$-$C_4$-Alkyithio, $C_1$-$C_4$-Halogenalkoxy, $C_1$-$C_4$-Halogenalkylthio, $C_1$-$C_4$-Alkylsulfonyl, Halogen, Cyano, Nitro, gegenbenenfalls substituiertes Phenyl oder gegebenenfalls substituiertes Phenoxy
m, p unabhängig voneinander 0, 1, 2 oder 3,
n 0 oder 1 bedeuten,
behandelt.

## Claims

1. A 2-substituted 2,3-epoxy-5-cyclohexenone derivative of the formula I

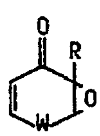

(I)

where W is C=O or $CHOR^1$, $R^1$ is hydrogen, $C_1$-$C_4$-alkyl, $C_2$-$C_4$-alkenyl, or $C_2$-$C_4$-alkylcarbonyl, R is a saturated or unsaturated hydrocarbon chain having 1 to 10 carbon atoms, which is unsubstituted or substituted by halogen, or the radical

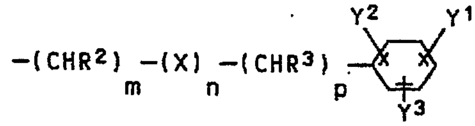

where $R^2$ and $R^3$ idenpendently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy, $C_1$-$C_4$-haloalkyl, $C_1$-$C_4$-alkylthio, $C_1$-$C_4$-haloalkoxy, $C_1$-$C_4$-haloalkylthio, $C_1$-$C_4$-alkylsulfonyl, halogen, cyano, nitro, unsubstituted or substituted phenyl or unsubstituted or substituted phenoxy, m and p independently of one another are each 0, 1, 2 or 3, and n is 0 or 1, with the exception of the compounds where W is C=O and R is methyl or 3-methyl-2-butenyl.

2. A 2-substituted 2,3-epoxy-5-cyclohexenone derivative of the formula I as claimed in claim 1, wherein W is C=O or $CHOR^1$, $R^1$ is hydrogen, R is

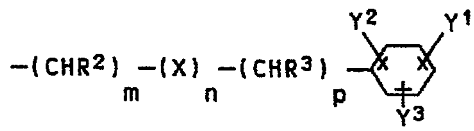

$R^2$ and $R^3$ independently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy $C_1$-$C_4$-haloalkyl, halogen or nitro, and m, n and p independently of one another are each 0 or 1.

3. 2,3-Epoxy-2-phenoxymethyl-5-cyclohexene-1,4-dione.

4. 2,3-Epoxy-2-(3-methyl-3-buten-1-ynyl)-5-cyclo-hexen-4-0l-1-one.

5. A fungicide containing an inert additive and a 2-substituted 2,3-epoxy-5-cylohexenone derivative I of the formula I

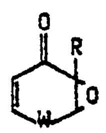 (I)

where W is C=O or $CHOR^1$, $R^1$ is hydrogen, $C_1$-$C_4$-alkyl, $C_2$-$C_4$-alkenyl, $C_2$-$C_4$-alkynyl or $C_2$-$C_4$-alkylcarbonyl, R is a saturated or unsaturated hydrocarbon chain having 1 to 10 carbon atoms, which is unsubstituted or substituted by halogen, or the radical

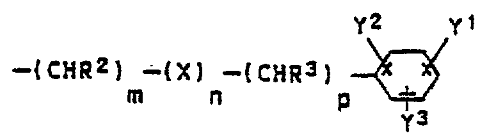

where $R^2$ and $R^3$ independently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy, $C_1$-$C_4$-haloalkyl, $C_1$-$C_4$-alkylthio, $C_1$-$C_4$-haloalkoxy, $C_1$-$C_4$-haloalkylthio, $C_1$-$C_4$-alkylsulfonyl, halogen, cyano, nitro, unsubstitued or substituted phenyl or unsubstituted or substituted phenoxy, m and p independently of one another are each 0, 1, 2 or 3 and n is 0 or 1.

6. A process for controlling fungi, wherein the fungi or the seeds, plants, materials or soil to be protected against fungus attack are treated with a 2-substituted 2,3-epoxy-5-cyclohexenone derivative I of the formula I

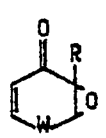 (I)

where W is C=O or $CHOR^1$, $R^1$ is hydrogen, $C_1$-$C_4$-alkyl, $C_2$-$C_4$-alkenyl, $C_2$-$C_4$-alkynyl or $C_2$-$C_4$-alkylcarbonyl, R is a saturated or unsaturated hydrocarbon chain having 1 to 10 carbon atoms, which in unsubstituted or substituted by halogen, or the radical

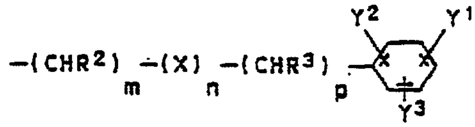

where $R^2$ and $R^3$ independently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy, $C_1$-$C_4$-haloalkyl, $C_1$-$C_4$-alkylthio, $C_1$-$C_4$-haloalkoxy, $C_1$-$C_4$-haloalkylthio, $C_1$-$C_4$-alkylsulfonyl, halogen, cyano, nitro, unsubstituted or substituted phenyl or unsubstituted or substituted phenoxy, m and p independently of one another are each 0, 1, 2 or 3 and n is 0 or 1.

7. A herbicide containing an inert additive and a 2-substituted 2,3-epoxy-5-cyclohexenone derivative of the formula I

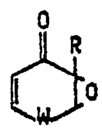

(I)

where W is C=O or $CHOR^1$, $R^1$ is hydrogen, $C_1$-$C_4$-alkyl, $C_2$-$C_4$-alkenyl, $C_2$-$C_4$-alkynyl or $C_2$-$C_4$-alkylcarbonyl, R is a saturated or unsaturated hydrocarbon chain having 1 to 10 carbon atoms, which is unsubstituted or substituted by halogen, or the radical

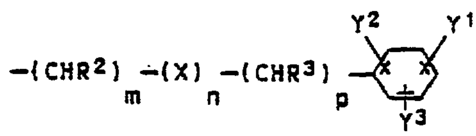

where $R^2$ and $R^3$ independently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy, $C_1$-$C_4$-haloalkyl, $C_1$-$C_4$-alkylthio, $C_1$-$C_4$-haloalkoxy, $C_1$-$C_4$-haloalkylthio, $C_1$-$C_4$-alkylsulfonyl, halogen, cyano, nitro, unsubstituted or substituted phenyl or unsubstituted or substituted phenoxy, m and p idependently of one another are each 0, 1, 2 or 3 and is 0 or 1.

8. A process for controlling the growth of undesirable plants, wherein the plants or the soil to be kept free from undesirable plant growth are treated with a 2-substituted 2,3-epoxy-5-cyclohexenone derivative I of the formula I

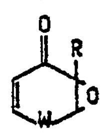

(I)

where W is C=O or $CHOR^1$, $R^1$ is hydrogen, $C_1$-$C_4$-alkyl, $C_2$-$C_4$-alkenyl, $C_2$-$C_4$-alkynyl or $C_2$-$C_4$-alkylcarbonyl, R is a saturated or unsaturated hydrocarbon chain having 1 to 10 carbon atoms, which is unsubstituted or substituted by halogen, or the radical

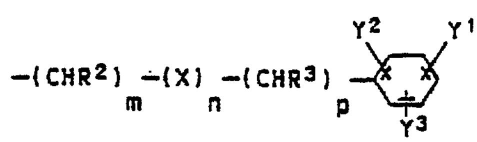

where $R^2$ and $R^3$ independently of one another are each hydrogen or methyl, X is oxygen or sulfur, $Y^1$, $Y^2$ and $Y^3$ are hydrogen, $C_1$-$C_4$-alkyl, $C_1$-$C_4$-alkoxy, $C_1$-$C_4$-haloalkyl, $C_1$-$C_4$-alkylthio, $C_1$-$C_4$-haloalkoxy, $C_1$-$C_4$-haloalkylthio, $C_1$-$C_4$-alkylsulfonyl, halogen, cyano, nitro, unsubstituted or substituted phenyl or unsubstituted or substituted phenoxy, m and p independently of one another are each 0, 1, 2 or 3 and n is 0 or 1.

## Revendications

1. Dérivés de la 2,3-époxy-5-cyclohexénone substitués en position 2 et répondant à la formule I

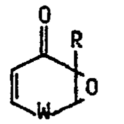

(I)

dans laquelle
W représente C=O ou $CHOR^1$,
$R^1$ représente l'hydrogène, un groupe alkyle en $C_1$-$C_4$, alcényle en $C_2$-$C_4$, alcynyle en $C_2$-$C_4$ ou alkylcarbonyle en $C_2$-$C_4$, R représente une chaîne hydrocarbonée saturée ou insuturée en $C_1$-$C_{10}$ éventuellement substituée par des halogènes, ou le groupe

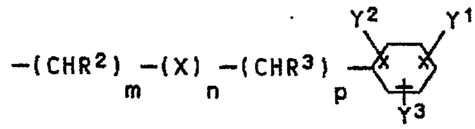

$R^2$, $R^3$ représentent chacun, indépendamment l'un de l'autre, l'hydrogène ou un groupe méthyle,
X représente l'oxygène ou le souffre,
$Y^1$, $Y^2$, $Y^3$ représentent l'hydrogène, des groupes alkyle en $C_1$-$C_4$, alkylthio en $C_1$-$C_4$, halogénoalkylthio en $C_1$-$C_4$, alkylsulfonyle en $C_1$-$C_4$, des halogènes des groupes cyano, nitro, phényle éventuellement substitué ou phénoxy éventuellement substitué,
m, p sont égaux chacun, indépendamment l'un de l'autre, à 0, 1, 2 ou 3,
n est égal à 0 ou 1, à l'exclusion des composés pour lesquels
W représente le groupe C=O et R représente un groupe méthyle ou 3-méthyl-2-buténylе.

2. Dérivés de la 2,3-époxy-5-cyclohexénone substitués en position 2, de formule I selon la revendication 1, caractérisés par le fait que
w représente C=O ou $CHOR^1$,
$R^1$ représente l'hydrogène,

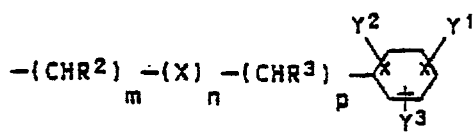

$R^2$, $R^3$ représentent chacun, indépendamment l'un de l'autre, l'hydrogène ou un groupe méthyle,
X représente l'oxygène ou le souffre,
$Y^1$, $Y^2$, $Y^3$ représentent l'hydrogène, des groupes alkyle en $C_1$-$C_4$, alcoxy en $C_1$-$C_4$, halogénoalkyle en $C_1$-$C_4$, des halogènes ou des groupes nitro,
m, n et p sont égaux chacun, indépendamment les uns des autres, à 0 ou 1.

3. La 2,3 époxy-2-phénoxyméthyl-5-cyclohexène-1,4-dione.

4. La 2,3-époxy-2-(3-méthyl-3-butène-1-inyl)-5-cyclo-hexène-4-0l-1one.

5. Produit fongicide contenant un additif inerte et un dérivé de la 2,3-époxy-5-cyclohexénone substitué en position 2 et répondant à la formule

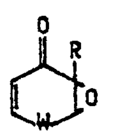

(I)

dans laquelle
W représente C=O ou $CHOR^1$,
$R^1$ représente l'hydrogène, un groupe alkyle en $C_1$-$C_4$, alcényle en $C_2$-$C_4$, alcynyle en $C_2$-$C_4$ ou alkylcarbonyle en $C_2$-$C_4$,
R représente une chaîne hydrocarbonée saturée ou insaturée contenant 1 à 10 atomes de carbone, qui est éventuellement substituée par des halogènes, ou le groupe

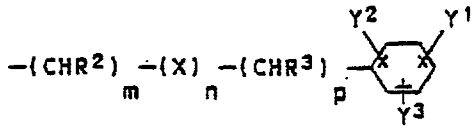

$R^2$, $R^3$ représentent chacun, indépendamment l'un de l'autre, l'hydrogène ou un groupe méthyle,
X représente l'oxygène ou le soufre,
$Y^1$, $Y^2$, $Y^3$ représentent l'hydrogène, des groupes alkyle en $C_1$-$C_4$, alcoxy en $C_1$-$C_4$, halogénoalkyle en $C_1$-$C_4$, alkylthio en $C_1$-$C_4$, halogénoalcoxy en $C_1$-$C_4$, halogénoalkylthio en $C_1$-$C_4$, alkylsulfonyle en $C_1$-$C_4$, des halogènes, des groupes cyano, nitro, phényle éventuellement substitué ou phénoxy éventuellement substitué,
m, p sont égaux chacun, indépendamment l'un de l'autre, à 0, 1, 2 ou 3,
n est égal à 0 ou 1.

6. Procédé pour combattre les mycètes, caractérisé en ce que l'on traite les mycètes ou les semences, plantes, matières ou le sol à protéger contre l'attaque par les mycètes par un dérivé de la 2,3-époxy-5-cyclohexénone substitué en position 2, de formule I

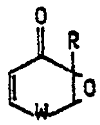

(I)

dans laquelle W représente C=O ou $CHOR^1$, $R^1$ représente l'hydrogène, un groupe alkyle en $C_1$–$C_4$, alcényle en $C_2$-$C_4$, alcynyle en $C_2$-$C_4$ ou alkylcarbonyle en $C_2$-$C_4$, R représente une chaîne hydrocarbonée saturée ou insaturée contenant 1 à 10 atomes de carbone, qui est éventuellement substituée par des halogènes, ou le groupe

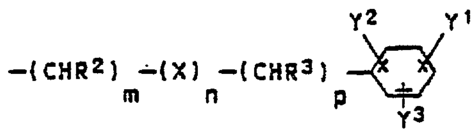

$R^2$, $R^3$ représentent chacun, indépendamment l'un de l'autre, l'hydrogène ou un groupe méthyle,
X représente l'oxygène ou le soufre,
$Y^1$, $Y^2$, $Y^3$ représentent l'hydrogène, des groupes alkyle en $C_1$-$C_4$, alcoxy en $C_1$–$C_4$, halogénoalkyle en $C_1$–$C_4$, alkylthio en $C_1$-$C_4$, halogénoalcoxy en $C_1$–$C_4$, halogénoalkylthio en $C_1$–$C_4$, alkylsulfonyle en $C_1$–$C_4$, des halogènes, des groupes cyano, nitro, phényle éventuellement substitué ou phénoxy éventuellement substitué,
m, p sont égaux chacun, indépendamment l'un de l'autre, à 0, 1, 2 ou 3,
n est égal à 0 ou 1.

7. Produit herbicide contenant un additif inerte et un dérivé de la 2,3-époxy-5-cyclohéxone substitué en position 2, répondant à la formule

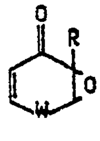

(I)

dans laquelle
W représente C=O ou $CHOR^1$,
$R^1$ représente l'hydrogène, un groupe alkyle en $C_1$–$C_4$, alcényle en $C_2$–$C_4$, alcynyle en $C_2$–$C_4$ ou alkylcarbonyle en $C_2$–$C_4$.
R représente une chaîne hydrocarbonée saturée ou insaturée contenant 1 à 10 atomes de carbone, qui est éventuellement substituée par des halogènes, ou le groupe

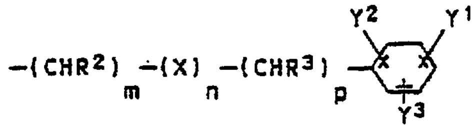

$R^2$, $R^3$ représentent chacun, indépendamment l'un de l'autre, l'hydrogène ou un groupe méthyle,
X représente l'oxygène ou le soufre,
$Y^1$, $Y^2$, $Y^3$ représentent l'hydrogène, les groupes alkyle en $C_1$–$C_4$, alcoxy en $C_1$–$C_4$, halogénoalkyle en $C_1$–$C_4$, alkylthio en $C_1$-$C_4$, halogénoalcoxy en $C_1$–$C_4$, halogénoalkylthio en $C_1$–$C_4$, alkylsulfonyle en $C_1$–$C_4$, des halogènes, des groupes cyano, nitro, phényle éventuellement substitué ou phénoxy éventuellement substitué,
m, p sont égaux chacun, indépendamment l'un de l'autre, à 0, 1, 2 ou 3,
n est égal à 0 ou 1.

8. Procédé pour combattre la croissance de végétaux indésirables,caractérisé par le fait que l'on traite les végétaux ou les terrains qu'on veut protéger contre les croissances de végétaux indésirables par un dérivé de la 2,3-époxy-5-cyclohexénone substitué en position 2, de formule I

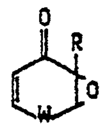

(I)

W représente C=O ou CHOR[1],
$R^1$ représente l'hydrogène, un groupe alkyle en $C_1$–$C_4$, alcényle en $C_2$–$C_4$, alcynyle en $C_2$–$C_4$ ou alkylcarbonyle en $C_2$–$C_4$,
R représente une chaîne hydrocarbonée saturée ou insaturée contenant 1 à 10 atomes de carbone, qui est éventuellement substituée par des halogènes, ou le groupe

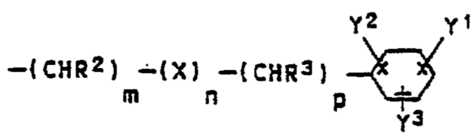

$R^2$, $R^3$ représentent chacun, indépendamment l'un de l'autre, l'hydrogène ou un groupe méthyle,
X représente l'oxygène ou le soufre,
$Y^1$, $Y^2$, $Y^3$ représentent l'hydrogène, les groupes alkyle en $C_1$–$C_4$, alcoxy en $C_1$–$C_4$, halogénoalkyle en $C_1$–$C_4$, alkylthio en $C_1$-$C_4$, halogénoalcoxy en $C_1$–$C_4$, halogénoalkylthio en $C_1$–$C_4$, alkylsulfonyle en $C_1$–$C_4$, des halogènes, des groupes cyano, nitro, phényle éventuellement substitué ou phénoxy éventuellement substitué,
m, p sont égaux chacun, indépendamment l'un de l'autre, à 0, 1, 2 ou 3,
n est égal à 0 ou 1.